# EUROPEAN PATENT SPECIFICATION

(11) **EP 3 651 660 B1**
(45) Date of publication and mention of the grant of the patent: **18.10.2023**
(21) Application number: 18831994.1
(22) Date of filing: 13.07.2018
(51) Int. Cl.: A61B 17/12, A61F 2/01, A61F 2/06, A61F 2/07, A61F 2/89, A61F 2/844

(54) **STENT GRAFTS AND METHODS OF ENHANCING FLEXIBILITY OF STENT GRAFTS BY THERMAL PLEATING**
STENTGRAFTS UND VERFAHREN ZUR ERHÖHUNG DER FLEXIBILITÄT VON STENTGRAFTS DURCH THERMISCHES FALTEN
ENDOPROTHÈSES COUVERTES ET PROCÉDÉS D'AMÉLIORATION DE LA FLEXIBILITÉ D'ENDOPROTHÈSES COUVERTES PAR PLISSAGE THERMIQUE

(30) Priority: 14.07.2017 US 201762532737 P
(43) Date of publication of application: 20.05.2020
(73) Proprietor: Endologix LLC, Irvine, CA 92618 (US)
(72) Inventor: EHNES, Dale, Forestville, California 95436 (US)
(74) Representative: J A Kemp LLP
(86) International application number: PCT/US2018/042158
(87) International publication number: WO 2019/014634

(56) References cited:
- EP-A1- 2 317 955
- EP-A1- 3 067 014
- WO-A1-2016/183128
- WO-A1-2016/183128
- US-A- 3 054 404
- US-A- 6 010 529
- US-A1- 2004 033 364
- US-A1- 2004 033 364
- US-A1- 2009 125 095
- US-A1- 2009 125 095
- US-A1- 2013 261 731
- US-B1- 6 814 748
- US-B1- 7 090 693
- US-B2- 7 862 609

## Description

### FIELD

One or more example embodiments described herein relate generally to stent grafts and methods of making and using stent grafts, and in specific embodiments, to stent grafts and methods of making stent grafts that are flexible.

### BACKGROUND

Aneurysms are enlargements or bulges in blood vessels that are often prone to rupture and which therefore present a serious risk to patients. Aneurysms may occur in any blood vessel but are of particular concern when they occur in the cerebral vasculature or an aorta.

Abdominal aortic aneurysms (AAA's) are classified based on their location within the aorta as well as their shape and complexity. Aneurysms that are found below the renal arteries are referred to as infrarenal abdominal aortic aneurysms. Suprarenal abdominal aortic aneurysms occur above the renal arteries. Thoracic aortic aneurysms (TAA's) occur in the ascending, transverse, or descending part of the upper aorta.

Stent grafts have come into widespread use for the treatment of aneurysms. Various stent grafts provide a graft layer to reestablish a flow lumen through an aneurysm as well as a stent structure to support the graft. In general, an endoluminal repair using a stent graft involves accessing an aneurysm endoluminally through either or both common iliac arteries. The stent graft is then implanted to treat the aneurysm.
EP 2 317 955 A1 discloses a medical device including a corrugated braid and an associated method. WO 2016/183128 A1 discloses a stent-graft which aims to provide improved flexibility. EP 3 067 014 A1 discloses a stent graft with a portal for a side branch device.

### SUMMARY OF THE DISCLOSURE

The invention is defined in claim 1. Further aspects and preferred embodiments are defined in the dependent claims. Any aspects, embodiments and examples of the present disclosure which do not fall under the scope of the appended claims do not form part of the invention and are merely provided for illustrative purposes.

In an embodiment, the applying of the heat may include applying an iron to creases of the pleats in the graft material.

In an embodiment, the iron may be heated between 320 °C to 390 °C prior to applying the iron to the creases.

In an embodiment, the compressing of the stent graft may include axially compressing, and optionally circularly compressing the stent graft.

In an embodiment, the applying of the heat may include baking the stent graft in an oven for a predetermined time period after forming the pleats.

In an embodiment, the oven may be heated to 320 °C prior to baking the stent graft.

In an embodiment, the predetermined time period may be 5 minutes.

In an embodiment, the predetermined time period may be greater than 5 minutes.

In an embodiment, the forming of the pleats may include folding the graft material over an adjacent portion of the graft material.

In an embodiment, the folding of the graft material may include folding the graft material abluminally with respect to a lumen of the stent graft.

In an embodiment, the folding of the graft material may include folding the graft material adluminally with respect to a lumen of the stent graft.

In an embodiment, the forming of the pleats may include folding the graft material adluminally with respect to a lumen of the stent graft at a portion of the graft material, and folding the graft material abluminally with respect to the lumen of the stent graft at a different portion of the graft material.

In an embodiment, the forming of the pleats may include folding the graft material over an adjacent portion of the graft material to have a graft space between adjacent stent members that is greater on a greater curvature side of the stent graft than on a lesser curvature side of the stent graft.

In an embodiment, the method may further include increasing the graft space on the greater curvature side to decrease a radius of curvature of the stent graft.

Another implementation of the present disclosure is a stent graft including a graft formed of graft material, and a stent attached to the graft and including stent members. The graft material is folded between the stent members over an adjacent portion of the graft members to form a pleat, and the pleat has a graft space between corresponding stent members on a greater curvature side of the stent graft that is greater than on a lesser curvature side of the stent graft.

The invention also relates to a stent graft manufactured by a process comprising the method of any of claims 1 to 14.

### BRIEF DESCRIPTION OF THE DRAWINGS

FIG. 1 shows a stent graft in accordance with an embodiment prior to thermal setting of pleats.
FIG. 2A is a flowchart of a method in accordance with an embodiment.
FIGS. 2B and 2C show methods that can be used with the method of FIG. 2A in accordance with various embodiments.
FIG. 3 shows a stent graft in an axially compressed state in accordance with an embodiment.
FIG. 4 shows an iron being used on graft material of a graft member of a stent graft in accordance with an embodiment to thermally lock in pleats in the graft material.
FIG. 5 shows a stent graft in accordance with an embodiment in a longitudinally extended state after pleats in graft material have been thermally set.
FIG. 6 shows a stent graft in accordance with an embodiment in a bent configuration after pleats in a graft member have been thermally set to form pleated sections in the stent graft.
FIG. 7A shows a stent graft in accordance with an embodiment having pleated sections that are tucked abluminally.
FIG. 7B shows an inner surface of a graft member of the stent graft of FIG. 7A.
FIG. 8A shows a stent graft in accordance with an embodiment having pleated sections that are tucked adluminally.
FIG. 8B shows an inner surface of a graft member of the stent graft of FIG. 8A.
FIG. 9A shows a stent graft in accordance with an embodiment having pleated sections that are tucked adluminally on a proximal end and abluminally on a distal end of the stent graft.
FIG. 9B shows an inner surface of a graft member of the stent graft of FIG. 9A.
FIG. 10 shows two embodiments of a stent graft to illustrate an effect of graft spacing between stent crowns on an achievable radius of curvature.
FIG. 11 shows an embodiment of a stent graft in a circularly compressed configuration.

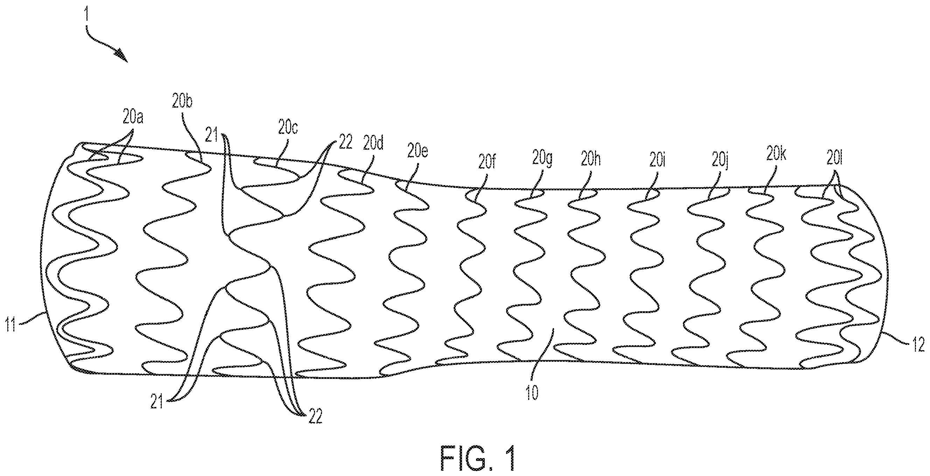

The invention relates to a method for forming pleats in a stent graft and to a stent graft as illustrated in fig. 1-10.

### DETAILED DESCRIPTION

In the following detailed description, reference is made to the accompanying drawings, which form a part of this specification. In the drawings, similar symbols typically identify similar items, unless context dictates otherwise.

Various embodiments provide for an enhancement in the flexibility of stent grafts by thermal pleating the stent grafts. Various embodiments provide for endovascular stent graft flexibility enhancements by (1) manually pleating the graft material to nest the geometry of the stent graft in a preferred orientation, such as by axially compressing the stent graft; and (2) thermally treating the stent graft while in the compressed position so as to preferentially lock in the pleats to give the graft material a thermal memory to retain flexibility once the graft is extended again in length. Various embodiments allow for improving the flexibility of stent graft systems for vasculature applications by having pleats in the graft material that are thermally set.

Various embodiments provide for thermally locking in a preferable pleat geometry in a graft material of a stent graft. Such a thermally locked in pleat geometry for the graft material may allow, for example, each crown formed by a stent member of a stent that has a zig pattern within the graft material to consistently move relative to zigs of adjacent stent members without generating significant shear forces within the graft material even if the stent members are fully fused or sintered within the graft material. In various embodiments, thermally set pleats effectively reduce or minimize the randomness of crowns contacting to allow the crowns to uniformly tuck underneath or go over adjacent crowns as desired.

Various methods in accordance with various embodiments are provided herein for thermally locking in a preferable pleat geometry. One such method in accordance with various embodiments includes thermally ironing the pleats. In some such embodiments, the stent graft is initially axially compressed to produce a desired pleating pattern. In some embodiments, a soldering iron set to a temperature of, for example, 320 °C to 390 °C is then used to wipe the pleated areas between adjacent stent members with the tip or barrel of the iron to thermally lock in the fold or pleat in the graft material. In various embodiments, other suitable temperatures can be used for the iron for ironing the pleats. In various embodiments, once each of the folds or pleats are thermally locked at the desired areas in the graft material, the stent graft is axially pulled back to its native length and will have flexibility that is improved (or greatly improved) over a non-pleated stent graft.

Another method in accordance with various embodiments for thermally locking in a preferable pleat geometry includes thermally baking the pleats. In some such embodiments, the stent graft is initially axially compressed to produce a desired pleating pattern. Also, in some such embodiments, the pleated part or parts of the stent graft is then placed in an oven set to a temperature of, for example, 320 °C or any other suitable temperature for a desired or predetermined time to thermally lock in the folds or pleats. In various embodiments the baking times in the oven are, for example, 5 minutes, 10 minutes, 20 minutes, and/or the like. However, the present disclosure is not limited thereto, and in other embodiments, other suitable baking times can be used. In some embodiments, 5 minutes may be preferable as a short amount of time to effectively lock in the pleats. In various embodiments, the baking method may be advantageous in that it is a non-contact method for producing the pleats. In various embodiments, once the folds or pleats are thermally locked at the desired areas in the graft material, the stent graft is axially pulled back to its native length and will have flexibility that is improved (or greatly improved) over a non-pleated stent graft.

Referring now to FIG. 1, a stent graft 1 is shown, in accordance with an example embodiment. The stent graft 1 is a hollow tubular device having a graft member 10 forming a tubular wall with a proximal end 11 and a distal end 12 and defining an open lumen between the proximal end 11 and the distal end 12. While stent graft 1 as seen in FIG. 1 is depicted as being substantially tubular, it should be understood that stent graft 1 may be of any shape that is suitable for delivery to and placement in a target site of a patient. For example, portions of graft member 10 at either or both of proximal and distal ends 11 and 12 may be flared (inwardly or outwardly) or tapered (inwardly or outwardly). Furthermore, portions of graft member 10 may also include non-straight tubular portions, such as flared (inwardly or outwardly) portions, portions having bends or curvature, fenestrations, channels, bifurcated portions, and/or the like. Moreover, while the proximal and distal ends 11 and 12 are depicted as having a single open lumen, the present disclosure is not limited thereto. For example, one or both proximal end 11 and distal end 12 may be multi-lumen ends such as, for example, bifurcated open ends.

FIG. 1 shows the stent graft 1 in a longitudinally extended state prior to pleating. The stent graft 1 includes the graft member 10, and also includes stent members 20a, 20b, 20c, 20d, 20e, 20f, 20g, 20h, 20i, 20j, 20k, and 20l. In some embodiments, the stent members 20a, 20b, 20c, 20d, 20e, 20f, 20g, 20h, 20i, 20j, 20k, and 20l are connected to each other as a single stent, while in other embodiments they are separate from each other. In various embodiments, each of the stent members 20a, 20b, 20c, 20d, 20e, 20f, 20g, 20h, 20i, 20j, 20k, and 20l is made of undulating wires that are wound circumferentially along an axis in an open tubular configuration. The circumferentially wound undulating wire(s) may be circular or helical. Also, the circumferentially wound undulating wires may form zigs with peaks and valleys. For example, the stent member 20c is depicted as having a plurality of peaks 21 pointing towards the proximal end 11 of the stent graft 1 and a plurality of valleys 22 pointing towards the distal end 12 of the stent graft 1. In various embodiments, each of the stent members 20a, 20b, 20c, 20d, 20e, 20f, 20g, 20h, 20i, 20j, 20k, and 20l forms a crown with a plurality of peaks and valleys.

Each of the stent members 20a, 20b, 20c, 20d, 20e, 20f, 20g, 20h, 20i, 20j, 20k, and 20l may be made, for example, from stainless steel, a nickel titanium alloy (NiTi) such as NITINOL, or any other suitable material, including, but not limited to, a cobalt-based alloy such as ELGILOY, platinum, gold, titanium, tantalum, niobium, and/or combinations thereof. Each of the stent members 20a, 20b, 20c, 20d, 20e, 20f, 20g, 20h, 20i, 20j, 20k, and 20l may be balloon-expandable or self-expandable. In various embodiments, more than one stent member may be disposed at or near the proximal end 11 of the stent graft 1, such as two stent members as shown by the stent members 20a. Also, in various embodiments, more than one stent member may be disposed at or near the distal end 12 of the stent graft 1, such as two stent members as shown by the stent members 20l. While the embodiment in FIG. 1 shows a particular number of stent members, it should be appreciated that, in various embodiments, any suitable number of stent members may be used.

In some embodiments, the stent members 20a, 20b, 20c, 20d, 20e, 20f, 20g, 20h, 20i, 20j, 20k, and 20l are attached to or laminated within the graft member 10. In some embodiments, the graft member 10 extends from the proximal end 11 to the distal end 12. In some other embodiments, the graft member 10 does not cover the entire length of stent graft 1, and may, for example, leave the proximal end 11, the distal end 12, or both uncovered. In various embodiments, the stent members 20a, 20b, 20c, 20d, 20e, 20f, 20g, 20h, 20i, 20j, 20k, and 20l are fully laminated or fused within the graft member 10. In this case, the possibility of graft material wear for the graft member 10 may be reduced, which is a function of relative motion between the components. In various embodiments, the stent members 20a, 20b, 20c, 20d, 20e, 20f, 20g, 20h, 20i, 20j, 20k, and 20l are partially laminated, tethered, or free-floating within the graft member 10.

In various embodiments, the graft member 10 comprises graft material that is made from one or more polymers or other suitable materials. In some embodiments, the graft member 10 is made of polytetrafluoroethylene (ePTFE). In some embodiments, the graft member 10 is made of expanded polytetrafluoroethylene (ePTFE). In yet some other embodiments, the stent graft 1 may include at least one additional polymer layer, such as a drug eluting layer, for eluting a bioactive agent from the stent graft 1 after implantation.

In some embodiments, the stent graft 1 can be longitudinally compressed to form a plurality of circumferential pleats with a predetermined orientation. In some embodiments, the stent graft 1 can be longitudinally compressed to form a continuous helical pleat in the case of a continuously wound wire. In various embodiments, each pleat involves a creased or folded surface of the graft material of the graft member 10, typically formed in areas of the graft member 10 between the locations of the stent members 20a, 20b, 20c, 20d, 20e, 20f, 20g, 20h, 20i, 20j, 20k, and 20l. Each portion of the stent graft 1 between two adjacent pleats is referred to herein as a pleated section of the stent graft 1. In various embodiments, each of a plurality of circumferential pleats is disposed between the crowns formed by the stent members 20a, 20b, 20c, 20d, 20e, 20f, 20g, 20h, 20i, 20j, 20k, and 20l.

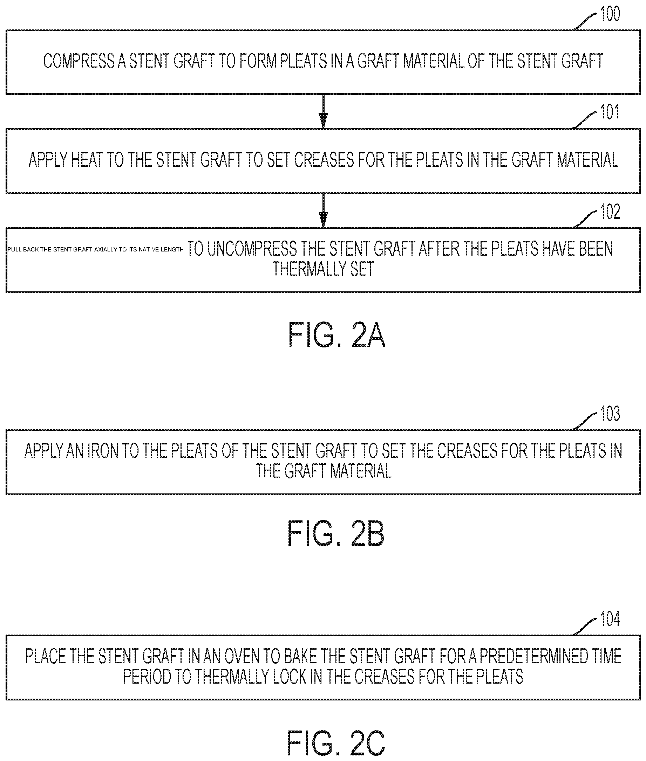

FIG. 2A is a flowchart of a method in accordance with an embodiment. FIGS. 2B and 2C show methods that can be used with the method of FIG. 2A. With reference to FIG. 2A, in step 100 a stent graft is compressed (axially and optionally circularly) to form pleats in a graft material of the stent graft. In step 101, heat is applied to the stent graft to set creases for the pleats in the graft material. In step 102, the stent graft is axially pulled back to its native length so as to uncompress the stent graft after the pleats have been thermally set. With reference to FIGS. 2A and 2B, in various embodiments the applying heat of step 101 is performed as in step 103 by applying an iron to the pleats of the stent graft to set the creases for the pleats in the graft material. With reference to FIGS. 2A and 2C, in various embodiments the applying heat of step 101 is performed as in step 104 by placing the stent graft in an oven to bake the stent graft for a predetermined time period to thermally lock in the pleats.

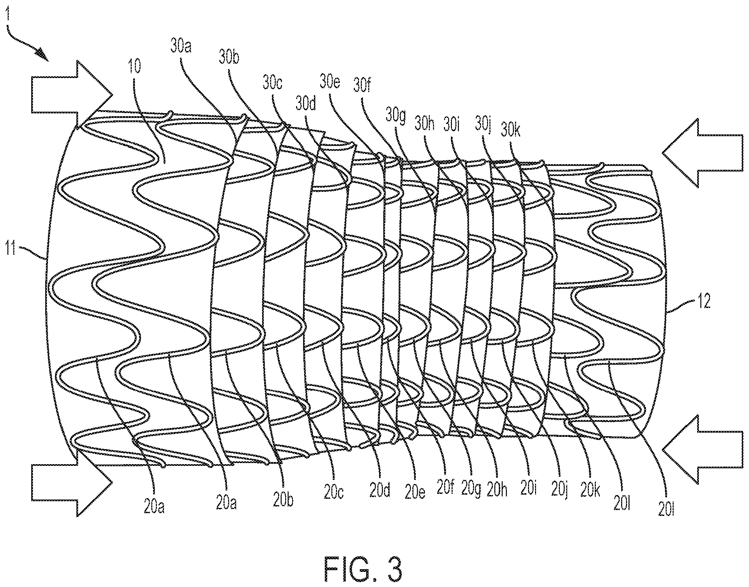

FIG. 3 shows the stent graft 1 in an axially compressed state in accordance with an embodiment. With reference to FIGS. 1, 2A, and 3, step 100 of axially compressing the stent graft 1 in FIG. 1 can result in the axially compressed version of the stent graft 1 as shown in FIG. 3, according to an embodiment. In various embodiments, the stent graft 1 is axially compressed by bringing the distal end 12 closer to the proximal end 11, which creates pleats 30a, 30b, 30c, 30d, 30e, 30f, 30g, 30h, 30i, 30j, and 30k where the graft material of the graft member 10 creases between the stent members 20a, 20b, 20c, 20d, 20e, 20f, 20g, 20h, 20i, 20j, 20k, and 20l, such that the stent members 20a, 20b, 20c, 20d, 20e, 20f, 20g, 20h, 20i, 20j, 20k, and 20l at least partially overlaps with (e.g., passes over or under) corresponding adjacent stent members.

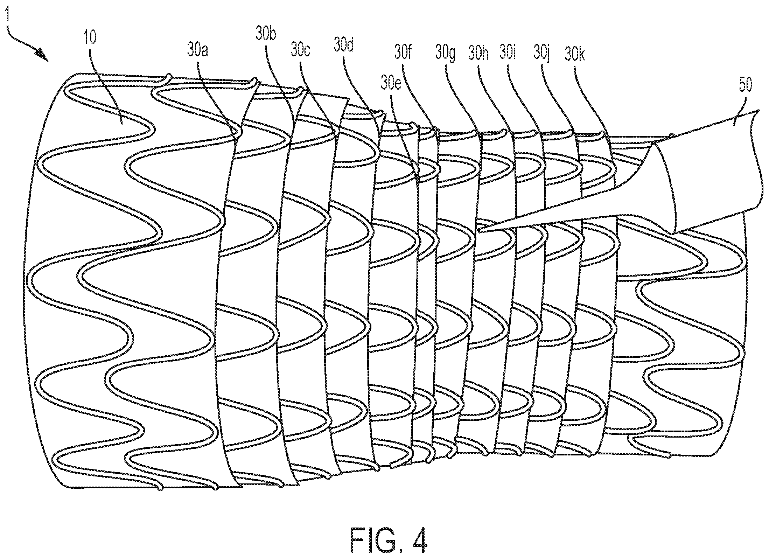

FIG. 4 shows an iron 50 being used on the graft material of the graft member 10 of the stent graft 1 in accordance with an embodiment to thermally lock in the pleats 30a, 30b, 30c, 30d, 30e, 30f, 30g, 30h, 30i, 30j, and 30k. With reference to FIGS. 2A, 2B, and 4, in various embodiments the step 101 of applying heat to the stent graft 1 includes the step 103 of applying the iron 50 to each of the pleats 30a, 30b, 30c, 30d, 30e, 30f, 30g, 30h, 30i, 30j, and 30k of the stent graft 1 to set the creases for the pleats 30a, 30b, 30c, 30d, 30e, 30f, 30g, 30h, 30i, 30j, and 30k in the graft material of the graft member 10. In various embodiments, the iron 50 is applied to (e.g., wiped over) each of the pleats 30a, 30b, 30c, 30d, 30e, 30f, 30g, 30h, 30i, 30j, and 30k around a circumference of the stent graft 1. In various embodiments, the temperature of the iron 50 is set to a temperature between 320 °C and 390 °C, for example. In various other embodiments, other suitable temperatures are used for the iron 50.

Additionally and/or alternatively, the manually compressed stent graft 1 as seen in FIG. 3 may be heated evenly or substantially evenly to set the pleats 30a, 30b, 30c, 30d, 30e, 30f, 30g, 30h, 30i, 30j, and 30k, such as, for example, by baking the stent graft 1 in an oven as shown in step 104 of FIG. 2C. With reference to FIGS. 2C and 3, the temperature of the oven may be set based on the baking time and the thickness of the graft member 10, and may be, for example, about 280 °C - 300 °C, 300 °C - 320 °C, 320 °C - 340 °C, 340 °C - 360 °C, or within any other suitable temperature range. The baking time may be, for example, about 5-10 minutes, 10-15 minutes, 15-20 minutes, 20-25 minutes, 25-30 minutes, or any other suitable time range for setting the pleats 30a, 30b, 30c, 30d, 30e, 30f, 30g, 30h, 30i, 30j, and 30k. In various example embodiments, three manually compressed stent grafts are placed in an oven of 320 °C for 5, 10, and 20 minutes, respectively. After baking, all three stent grafts are thermally set to the predetermined pleat orientation, and are able to retain the same orientation when compressed again naturally.

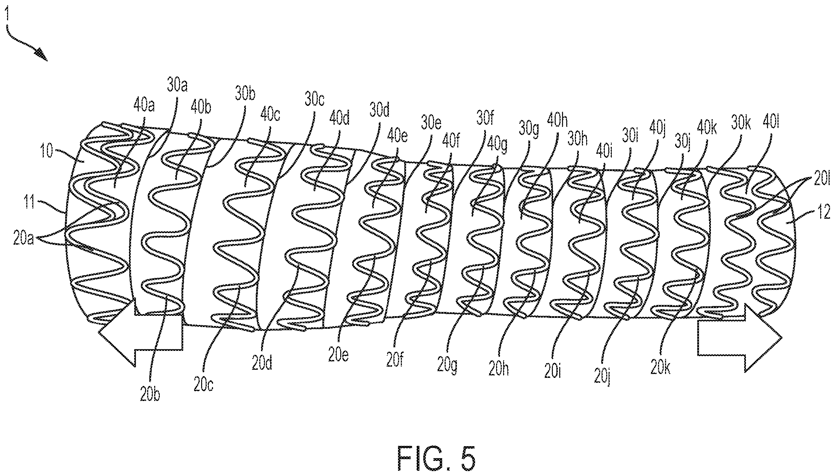

FIG. 5 shows the stent graft 1 in a longitudinally extended state after the pleats 30a, 30b, 30c, 30d, 30e, 30f, 30g, 30h, 30i, 30j, and 30k have been thermally set. In some embodiments, the pleats 30a, 30b, 30c, 30d, 30e, 30f, 30g, 30h, 30i, 30j, and 30k are a plurality of circumferential pleats. In some embodiments, the pleats 30a, 30b, 30c, 30d, 30e, 30f, 30g, 30h, 30i, 30j, and 30k form a continuous helical pleat. With reference to FIGS. 2A, 4, and 5, in various embodiments, the step 102 is performed on the stent graft 1 to pull (or extend) the stent graft 1 to uncompress the stent graft 1 from the axially compressed state as in FIG. 4 to the longitudinally extended state as in FIG. 5 after the pleats 30a, 30b, 30c, 30d, 30e, 30f, 30g, 30h, 30i, 30j, and 30k have been thermally set. The stent graft 1 in FIG. 5 is shown to include the pleated sections 40a, 40b, 40c, 40d, 40e, 40f, 40g, 40h, 40i, 40j, 40k, and 40l.

The pleated section 40a of the stent graft 1 includes the stent members 20a and a portion of the graft member 10 between the proximal end 11 and the pleat 30a. The pleated section 40b of the stent graft 1 includes the stent member 20b and a portion of the graft member 10 between the pleat 30a and the pleat 30b. The pleated section 40c of the stent graft 1 includes the stent member 20c and a portion of the graft member 10 between the pleat 30b and the pleat 30c. The pleated section 40d of the stent graft 1 includes the stent member 20d and a portion of the graft member 10 between the pleat 30c and the pleat 30d. The pleated section 40e of the stent graft 1 includes the stent member 20e and a portion of the graft member 10 between the pleat 30d and the pleat 30e. The pleated section 40f of the stent graft 1 includes the stent member 20f and a portion of the graft member 10 between the pleat 30e and the pleat 30f.

The pleated section 40g of the stent graft 1 includes the stent member 20g and a portion of the graft member 10 between the pleat 30f and the pleat 30g. The pleated section 40h of the stent graft 1 includes the stent member 20h and a portion of the graft member 10 between the pleat 30g and the pleat 30h. The pleated section 40i of the stent graft 1 includes the stent member 20i and a portion of the graft member 10 between the pleat 30h and the pleat 30i. The pleated section 40j of the stent graft 1 includes the stent member 20j and a portion of the graft member 10 between the pleat 30i and the pleat 30j. The pleated section 40k of the stent graft 1 includes the stent member 20k and a portion of the graft member 10 between the pleat 30j and the pleat 30k. The pleated section 40l of the stent graft 1 includes the stent members 20l and a portion of the graft member 10 between the pleat 30k and the distal end 12.

The stent graft 1 with predetermined or pre-set pleat orientation in accordance with various embodiments provides several advantages. Circumferential pleats provide space for longitudinal movement between stent members or crowns, thereby improving longitudinal flexibility during longitudinal compression and/or expansion, and improving radial flexibility as movement is permitted in the radial direction upon bending or even longitudinal compression. The pre-set pleats provide an advantage over random pleats because random pleats that are formed by compressing a stent graft, and especially those that protrude radially outward, tend to prevent longitudinal compression and generate internal forces within a stent graft that lead to kinking. Various embodiments disclosed herein overcome the problem of random pleating by predetermining and locking in the orientation of the pleats before the stent graft is longitudinally compressed (or uncompressed) or radially bent, thereby allowing the stent graft to automatically form a consistent and predetermined pleat orientation when radially bent or longitudinally compressed for loading, delivery, or implantation. A uniformed pleat orientation allows the stent members or crowns to consistently move relative to adjacent crowns without generating significant shear forces within the graft material.

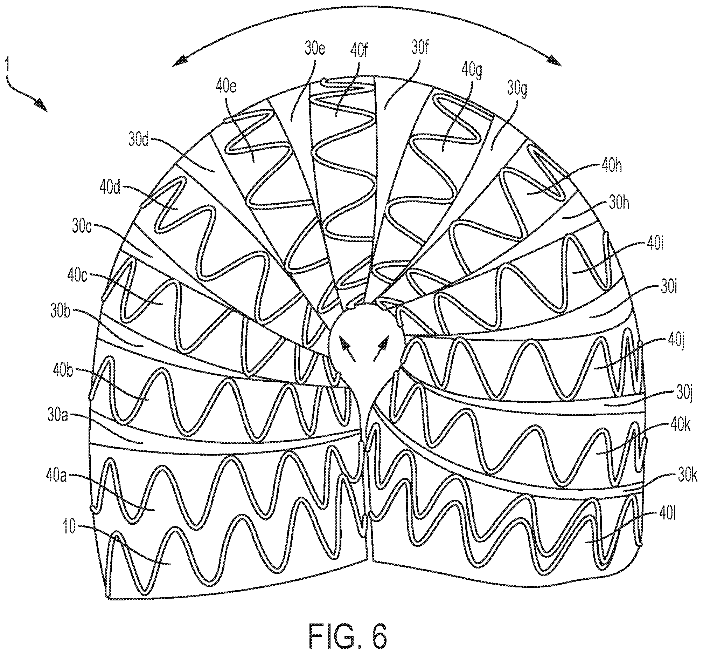

FIG. 6 shows the stent graft 1 in accordance with an embodiment in a bent configuration after the pleats 30a, 30b, 30c, 30d, 30e, 30f, 30g, 30h, 30i, 30j, and 30k in the graft member 10 have been thermally set to form the pleated sections 40a, 40b, 40c, 40d, 40e, 40f, 40g, 40h, 40i, 40j, 40k, and 40l in the stent graft 1. The stent graft 1 has improved kink resistance when bent due to the thermally set pleats. In some embodiments, the stent graft 1 is able to conform, for example, around a pin with a 0.325 inch diameter without kinking. In various embodiments, during bending, the pleats 30a, 30b, 30c, 30d, 30e, 30f, 30g, 30h, 30i, 30j, and 30k in the graft member 10 move under the bending force according to their preset orientation. This permits the stent graft 1 to bend about 180° or greater without having a substantial reduction in diameter in any portion of the bend. Thus, openness of a lumen of the stent graft 1 is maintained or substantially maintained, particularly in the area of the bend. This results in a higher-performing stent graft as blood flow is diminished only slightly, or not at all in the bend. In contrast, a stent graft without the preset pleats (e.g., circumferential or helical) may have a deformed portion when undergoing a similar degree of bending, where the deformed portion may cause a partial closing of a lumen resulting in suboptimal performance.

By longitudinally compressing a stent graft from a longitudinally extended configuration to a compressed configuration, pleats (e.g., a plurality of circumferential pleats or pleats forming a continuous helical pleat) of predetermined orientation can be formed such that the pleated sections nest within corresponding adjacent pleated sections along an axis. In various embodiments, circumferential pleats in any desired orientation may be pretreated thermally to lock the pleats in the desired orientation such that when the stent graft is longitudinally compressed again in a natural setting, for example, during stent graft loading, delivery, or implantation, the compressed stent graft will memorize and resume the preset pleat orientation. In various embodiments, a stent graft is manually compressed longitudinally to form a uniform abluminal pleat orientation. Each of the circumferential pleats is then ironed to set the creases in the graft member. After the ironing step, the stent graft is manually pulled back to its extended state. Such thermal pretreatment allows the stent graft to memorize the preset pleat orientation when being compressed again naturally. The pleats may also be pretreated thermally to form a uniform adluminal orientation, or a combination of abluminal and adluminal orientation, as desired.

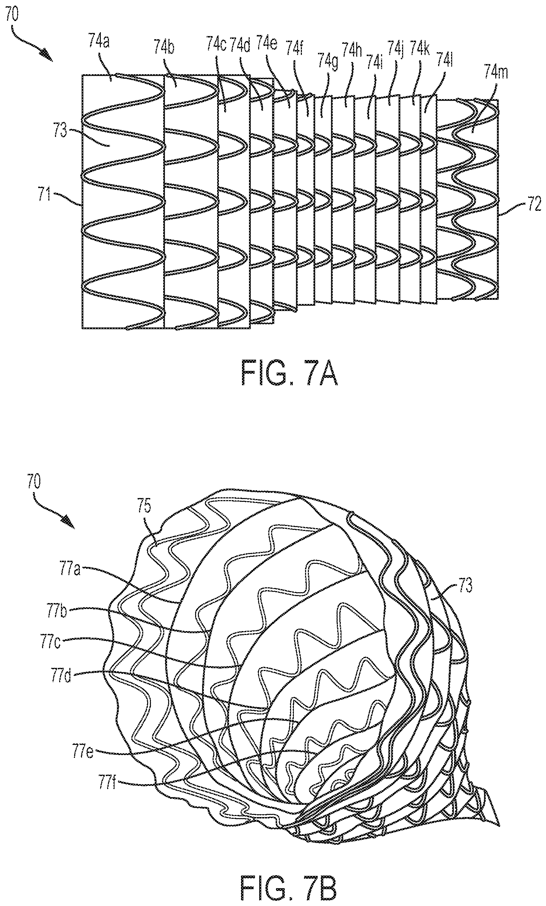

FIG. 7A shows a stent graft 70 in accordance with an embodiment including a graft member 73 with a proximal end 71 and a distal end 72 and having pleated sections 74a, 74b, 74c, 74d, 74e, 74f, 74g, 74h, 74i, 74j, 74k, 74l, and 74m that have zig valleys that are tucked abluminally with respect to adjacent zig peaks. The stent graft 70 includes the pleated sections 74a, 74b, 74c, 74d, 74e, 74f, 74g, 74h, 74i, 74j, 74k, 74l, and 74m that are preset through an intentional compression to be nested such that all of those pleated sections are tucked abluminally with respect to each other (that is, a more proximal pleated section is radially raised to cover a portion of its immediately distally adjacent pleated section). This causes the pleats to be formed in the stent graft 70 such that the crown valleys of the stent members are tucked abluminally with respect to each other. FIG. 7B shows an inner surface 75 of the graft member 73 of the stent graft 70 of FIG. 7A in accordance with an embodiment with pleats 77a, 77b, 77c, 77d, 77e, and 77f that form a "rough" inner surface due to the abluminal nesting orientation of the pleated sections 74a, 74b, 74c, 74d, 74e, 74f, 74g, 74h, 74i, 74j, 74k, 74l, and 74m.

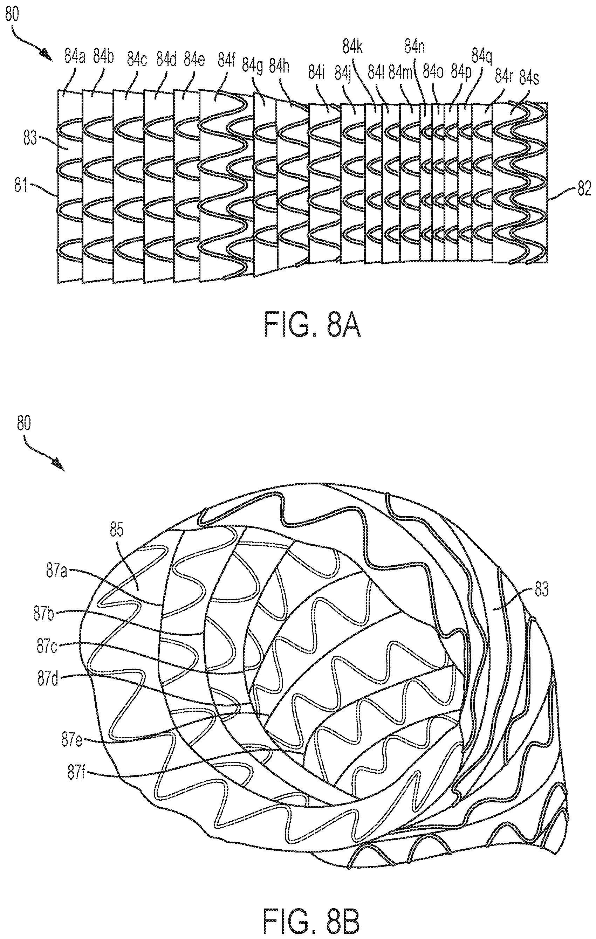

FIG. 8A shows a stent graft 80 in accordance with an embodiment including a graft member 83 with a proximal end 81 and a distal end 82 and having pleated sections 84a, 84b, 84c, 84d, 84e, 84f, 84g, 84h, 84i, 84j, 84k, 84l, 84m, 84n, 84o, 84p, 84q, 84r, and 84s that have zig valleys that are tucked adluminally with respect to adjacent zig peaks. The stent graft 80 includes the pleated sections 84a, 84b, 84c, 84d, 84e, 84f, 84g, 84h, 84i, 84j, 84k, 84l, 84m, 84n, 84o, 84p, 84q, 84r, and 84s that are preset through an intentional compression to be nested such that all of those pleated sections are tucked adluminally with respect to each other (that is, a more distal pleated section is radially raised to cover a portion of its immediately proximally adjacent pleated section). This causes the pleats to be formed in the stent graft 80 such that the crown valleys of the stent members are tucked adluminally with respect to each other to create reduced shear stress in the graft material. FIG. 8B shows an inner surface 85 of the graft member 83 of the stent graft 80 of FIG. 8A in accordance with an embodiment with pleats 87a, 87b, 87c, 87d, 87e, and 87f that form a "smooth" inner surface throughout the stent graft 80 due to the adluminal nesting orientation of the pleated sections.

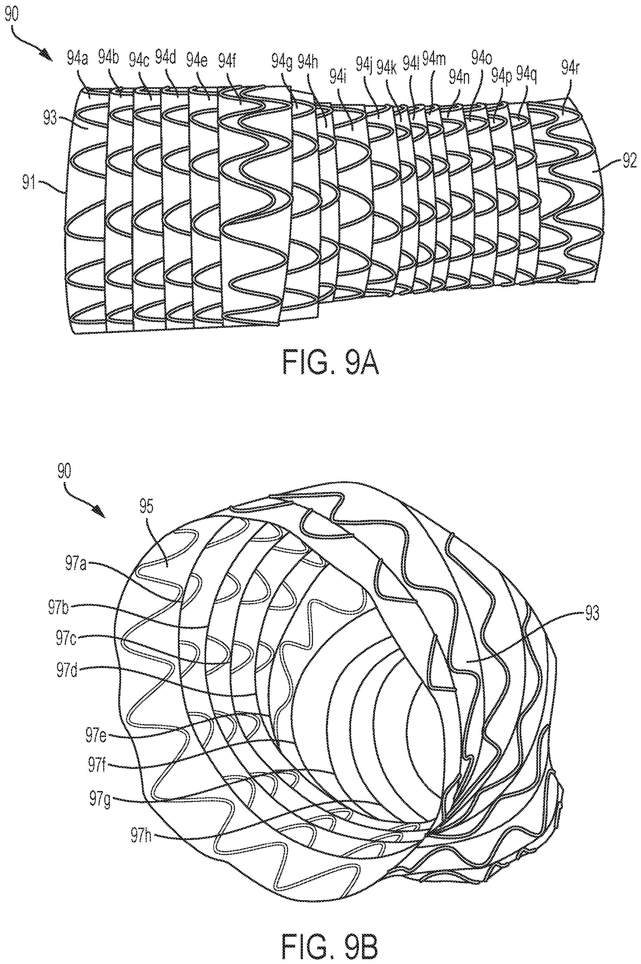

In yet some other embodiments, some of the pleated sections may be predetermined to be tucked adluminally, while others may be tucked abluminally. For example, in some embodiments, proximal pleated sections are tucked adluminally, while distal pleated sections are tucked abluminally. FIG. 9A shows a stent graft 90 in accordance with an embodiment including a graft member 93 with a proximal end 91 and a distal end 92 and having pleated sections 94a, 94b, 94c, 94d, 94e, 94f, 94g, 94h, 94i, 94j, 94k, 94l, 94m, 94n, 94o, 94p, 94q, and 94r. The stent graft 90 includes the pleated sections 94a, 94b, 94c, 94d, and 94e that are preset through an intentional compression to be nested such that all of those pleated sections are tucked adluminally with respect to each other. The stent graft 90 includes the pleated sections 94f, 94g, 94h, 94i, 94j, 94k, 94l, 94m, 94n, 94o, 94p, 94q, and 94r that are preset through an intentional compression to be nested such that all of those pleated sections are tucked abluminally with respect to each other. FIG. 9B shows an inner surface 95 of the graft member 93 of the stent graft 90 of FIG. 9A in accordance with an embodiment with pleats 97a, 97b, 97c, 97d, 97e, 97f, 97g, and 97h, where the pleats 97a, 97b, 97c, 97d, and 97e form a "smooth" inner surface due to the adluminal nesting orientation of the pleated sections 94a, 94b, 94c, 94d, 94e, and 94f, while the other pleats form a "rough" inner surface due to the abluminal nesting orientation of the remaining pleated sections. Various embodiments provide for a mechanical interlocking with modular components.

In various embodiments, a blood flow pattern in a stent graft may be controlled and modified by selecting the orientation of the pleats. For example, when the valleys of the stent crowns are tucked abluminally, the inner surface of the stent graft lumen is "rough" and may disrupt blood flow. On the other hand, when the valleys of the stent crowns are tucked adluminally, the inner surface of the stent graft lumen is "smooth," resulting in a desirable blood flow pattern and reduced shear stress on the graft material. Further, when the pleats are helical pleats, the inner surface of the stent graft lumen has a spiral pattern (which can have pleats that are tucked adluminally or abluminally), which may induce or maintain a desirable spiral blood flow pattern.

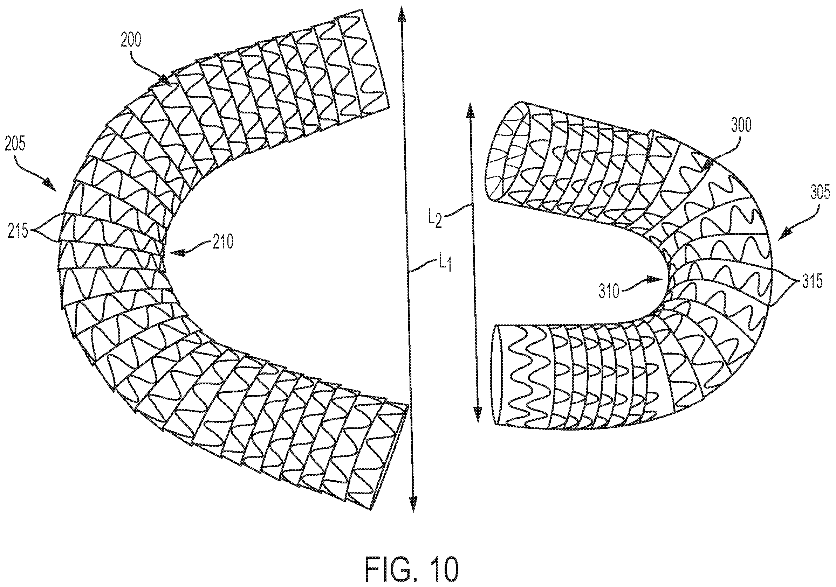

FIG. 10 illustrates the effect of graft spacing between stent members or crowns on an achievable radius of curvature. Each of stent graft 200 on the left and stent graft 300 on the right has a greater curvature side 205 and 305 and a lesser curvature side 210 and 310, respectively. The stent graft 200 on the left has a 2 mm graft space 215 between the crowns of the stent body at the greater curvature side 205, while stent graft 300 on the right has a 4 mm graft space 315 between the crowns of the stent body at the greater curvature side 305. Each of the stent graft 200 and the stent graft 300 has a smaller (or negative) graft space between the crowns of the stent body on the lesser curvature side 210 and 310 opposite the greater curvature side 205 and 305, respectively. The increased graft spacing on the greater curvature side 305 allows stent graft 300 to achieve a smaller radius of curvature when compared to the stent graft 200 due to the larger pleats which allow more nesting of the stent zig geometry. For example, the stent graft 200 has a curvature with an end-to-end length of L₁ (of about 125 mm), whereas the stent graft 300 has a curvature with an end-to-end length of L₂ (of about 80 mm), where L₁ is greater than L₂. In various embodiments, thermally pretreating a stent graft that has been longitudinally compressed allows for pleats to be formed that are consistent with a predetermined orientation. In various embodiments, each pleated section may include one crown or more than one crown, and pleated sections in different portions of the stent graft may include different numbers of crowns or otherwise have different width or spacing, to accommodate the shape of the stent graft, and to achieve a desired dimension and physical characteristics.

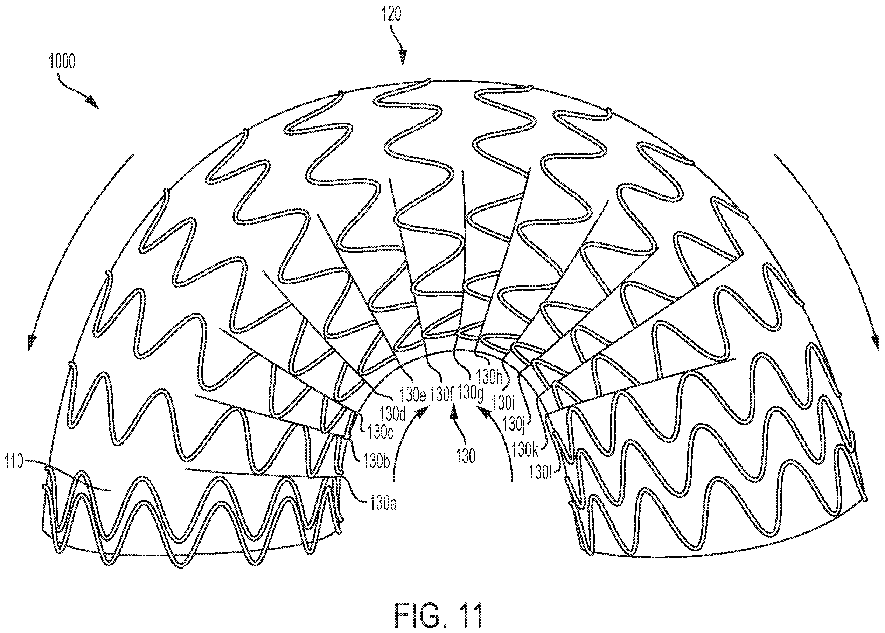

FIG. 11 shows a stent graft 1000 in accordance with an embodiment in a circularly compressed configuration. In the circularly compressed configuration, the stent graft 1000 with circular pleating can be formed. The stent graft 1000 with circular pleating has partial pleats 130a, 130b, 130c, 130d, 130e, 130f, 130g, 130h, 130i, 130j, 130k, and 130l that are thermally set in the graft member 110 on a lesser curvature side 130 of the stent graft 1000, but a greater curvature side 120 of the stent graft 1000 does not have pleats. For example, the partial pleats 130a, 130b, 130c, 130d, 130e, 130f, 130g, 130h, 130i, 130j, 130k, and 130l may be thermally set by applying heat to the partial pleats 130a, 130b, 130c, 130d, 130e, 130f, 130g, 130h, 130i, 130j, 130k, and 130l via an iron and/or baking the circularly compressed stent graft 1000 having the partial pleats. The stent graft 1000 with the circular pleating has improved conformability in a curved configuration with improved kink resistance. Thus, openness of a lumen of the stent graft 1000 is maintained or substantially maintained, while conforming to the curved configuration. This results in a higher-performing stent graft as blood flow is diminished only slightly, or not at all in the curved portion. In contrast, a stent graft without the preset circular pleating may have a deformed portion when undergoing a similar degree of curvature, where the deformed portion may cause a partial closing of a lumen resulting in suboptimal performance.

By circularly compressing a stent graft from a longitudinally extended configuration to a circularly compressed configuration, partial pleats (e.g., pleats on the lesser curvature and no pleats on the greater curvature of the graft material) of predetermined orientation can be formed such that the partial pleated sections nest within corresponding adjacent partial pleated sections along the curvature. In various embodiments, partial pleats in any desired orientation may be pretreated thermally to lock the partial pleats in one or more desired curved orientations such that when the stent graft is circularly compressed again in a natural setting, for example, during stent graft loading, delivery, or implantation, the compressed stent graft will memorize and resume the preset curved orientations. In various embodiments, a stent graft is manually compressed circularly to form a uniform abluminal partial pleat orientation. Each of the partial pleats is then thermally set by applying heat (e.g., by ironing or baking) to set the partial pleats in the graft member. After the thermal application step, the stent graft is manually pulled back to its extended state. Such thermal pretreatment allows the stent graft to memorize the preset partial pleat orientation when being circularly compressed again naturally. The partial pleats may also be pretreated thermally to form a uniform adluminal partial pleat orientation, or a combination of abluminal and adluminal partial pleat orientation, as desired.

In some embodiments, a stent graft may be formed to have circular pleating on a portion thereof, and axial pleating (e.g., circumferential or helical pleating) on another portion thereof. In this case, a portion of the stent graft may be circularly compressed to form the circular pleating as described with reference to FIG. 11, and another portion of the stent graft may be axially compressed to form the axial pleating as described with reference to FIG. 5. For example, such a stent graft may be particularly useful in a case of a descending thoracic where the upper portion of the aorta is curved (and thus, suited for receiving the circular pleated portion of the stent graft), while the descending section is relatively straight (and thus, suited for receiving the axial pleated portion of the stent graft). However, the present disclosure is not limited thereto, and it should be appreciated that according to various embodiments, a stent graft may include any number of circular pleated portions and axial pleated portions as needed or desired.

Various embodiments provide stent grafts with improved flexibility due to pleating that allows a nitinol zig pattern of stent members freedom to move over adluminally formed pleats. Various pleats are formed to create a preferential nesting pattern for maximum flexibility. In some embodiments, a portion of the pleating is abluminal (valley of nitinol crown for example) while the adjacent portion of the pleating is adluminal (peak of nitinol crown for example). In various embodiments, the stent is fully laminated or fused within the graft material, thereby reducing concerns about wear, since wear rate is a function of relative motion between components, and subsequent corrosion. In some embodiments, a fully laminated or fused stent within graft material has an advantage over embodiments where the stent is partially laminated, tethered, or free-floating within the graft material, since there can be less wear.

Various embodiments provide for producing pleats in a preferred directional orientation to create a smooth lumen in a stent graft. This allows for reducing or eliminating an undesirable random pleating in which there may be protrusions into the lumen in such a way that may disrupt blood flow. Various embodiments provide an advantage of improved kink resistance and improved flexibility. Various embodiments provide a fast method for locking in pleats. For example, in various embodiments, a non-contact oven pleating method allows for locking in pleats in as little as 5 minutes time.

The present invention is defined by the following claims.

## Claims

1. A method for forming pleats (30a-30k) in a stent graft (1, 70, 80, 90, 200, 300, 1000), comprising:
forming pleats (30a-30k) in a graft material of the stent graft (1, 70, 80, 90, 200, 300, 1000) by compressing the stent graft (1, 70, 80, 90, 200, 300, 1000);
applying heat to the stent graft (1, 70, 80, 90, 200, 300, 1000) to thermally set the pleats (30a-30k) in the graft material; and
**characterised by** comprising axially pulling back the stent graft to its native length to uncompress the stent graft (1, 70, 80, 90, 200, 300, 1000) after the pleats (30a-30k) are thermally set.

2. The method of claim 1, wherein the applying of the heat comprises applying an iron (50) to creases of the pleats (30a-30k) in the graft material.

3. The method of claim 2, wherein the iron (50) is heated between 320 °C to 390 °C prior to applying the iron (50) to the creases.

4. The method of any one of claims 1 to 3, wherein the compressing of the stent graft (1) includes axially compressing, and optionally circularly compressing, the stent graft (1, 70, 80, 90, 200, 300, 1000).

5. The method of either of claims 1 and 4, wherein the applying of the heat comprises baking the stent graft (1, 70, 80, 90, 200, 300, 1000) in an oven for a predetermined time period after forming the pleats (30a-30k).

6. The method of claim 5, wherein the oven is heated to 320 °C prior to baking the stent graft (1, 70, 80, 90, 200, 300, 1000).

7. The method of either of claims 5 and 6, wherein the predetermined time period is 5 minutes.

8. The method of either of claims 5 and 6, wherein the predetermined time period is greater than 5 minutes.

9. The method of any one of claims 1 to 8, wherein the forming of the pleats (30a-30k) comprises folding the graft material over an adjacent portion of the graft material.

10. The method of claim 9, wherein the folding of the graft material comprises folding the graft material abluminally with respect to a lumen of the stent graft (70).

11. The method of claim 10, wherein the folding of the graft material comprises folding the graft material adluminally with respect to a lumen of the stent graft (80).

12. The method of any one of claims 1 to 8, wherein the forming of the pleats (30a-30k) comprises:
folding the graft material adluminally with respect to a lumen of the stent graft (90) at a portion of the graft material; and
folding the graft material abluminally with respect to the lumen of the stent graft (90) at a different portion of the graft material.

13. The method of any one of claims 1 to 8, wherein the forming of the pleats (30a-30k) comprises folding the graft material over an adjacent portion of the graft material to have a graft space (215, 315) between adjacent stent members that is greater on a greater curvature side (205, 305) of the stent graft (200, 300) than on a lesser curvature side (210, 310) of the stent graft (200, 300).

14. The method of claim 13, further comprising increasing the graft space on the greater curvature side to decrease a radius of curvature of the stent graft (1).

15. A stent graft (1, 70, 80, 90, 200, 300, 1000) manufactured by a process comprising:
the method of any of claims 1 to 14.

## Patentansprüche

1. Verfahren zur Bildung von Falten (30a-30k) in einem Stenttransplantat (1, 70, 80, 90, 200, 300, 1000), umfassend:
Bilden von Falten (30a-30k) in einem Transplantatmaterial des Stenttransplantats (1, 70, 80, 90, 200, 300, 1000) durch Komprimieren des Stenttransplantats (1, 70, 80, 90, 200, 300, 1000);
Anwenden von Wärme auf das Stenttransplantat (1, 70, 80, 90, 200, 300, 1000), um die Falten (30a-30k) in dem Transplantatmaterial thermisch zu fixieren; und
**dadurch gekennzeichnet, dass** es das axiale Zurückziehen des Stenttransplantats auf seine ursprüngliche Länge umfasst, um das Stenttransplantat (1, 70, 80, 90, 200, 300, 1000) zu dekomprimieren, nachdem die Falten (30a-30k) thermisch verfestigt sind.

2. Verfahren nach Anspruch 1, wobei das Aufbringen der Wärme das Aufbringen eines Bügeleisens (50) auf Knicklinien der Falten (30a-30k) im Transplantatmaterial umfasst.

3. Verfahren nach Anspruch 2, wobei das Bügeleisen (50) vor dem Aufbringen des Bügeleisens (50) auf die Knicklinien auf zwischen 320 °C und 390 °C erhitzt wird.

4. Verfahren nach einem der Ansprüche 1 bis 3, wobei das Komprimieren des Stenttransplantats (1) ein axiales Komprimieren und optional ein kreisförmiges Komprimieren des Stenttransplantats (1, 70, 80, 90, 200, 300, 1000) enthält.

5. Verfahren nach einem der Ansprüche 1 und 4, wobei die Zufuhr von Wärme das Backen des Stenttransplantats (1, 70, 80, 90, 200, 300, 1000) in einem Ofen für eine vorbestimmte Zeitspanne nach dem Formen der Falten (30a-30k) umfasst.

6. Verfahren nach Anspruch 5, wobei der Ofen vor dem Backen des Stenttransplantats (1, 70, 80, 90, 200, 300, 1000) auf 320 °C erhitzt wird.

7. Verfahren nach einem der Ansprüche 5 und 6, wobei die vorbestimmte Zeitspanne 5 Minuten beträgt.

8. Verfahren nach einem der Ansprüche 5 und 6, wobei die vorbestimmte Zeitspanne größer als 5 Minuten ist.

9. Verfahren nach einem der Ansprüche 1 bis 8, wobei die Bildung der Falten (30a-30k) das Falten des Transplantatmaterials über einen benachbarten Abschnitt des Transplantatmaterials umfasst.

10. Verfahren nach Anspruch 9, wobei das Falten des Transplantatmaterials das Falten des Transplantatmaterials abluminal in Bezug auf ein Lumen des Stenttransplantats (70) umfasst.

11. Verfahren nach Anspruch 10, wobei das Falten des Transplantatmaterials das Falten des Transplantatmaterials adluminal in Bezug auf ein Lumen des Stenttransplantats (80) umfasst.

12. Verfahren nach einem der Ansprüche 1 bis 8, wobei das Formen der Falten (30a-30k) umfasst:
adluminales Falten des Transplantatmaterials in Bezug auf ein Lumen des Stenttransplantats (90) an einem Abschnitt des Transplantatmaterials; und
abluminales Falten des Transplantatmaterials in Bezug auf das Lumen des Stenttransplantats (90) an einem anderen Abschnitt des Transplantatmaterials.

13. Verfahren nach einem der Ansprüche 1 bis 8, wobei das Bilden der Falten (30a-30k) das Falten des Transplantatmaterials über einen benachbarten Abschnitt des Transplantatmaterials umfasst, um einen Transplantatraum (215, 315) zwischen benachbarten Stentgliedern aufzuweisen, der auf einer Seite (205, 305) mit größerer Krümmung des Stenttransplantats (200, 300) größer ist als auf einer Seite (210, 310) mit geringerer Krümmung des Stenttransplantats (200, 300).

14. Verfahren nach Anspruch 13, das ferner die Vergrößerung des Transplantatraums auf der Seite mit der größeren Krümmung umfasst, um den Krümmungsradius des Stenttransplantats (1) zu verringern.

15. Stenttransplantat (1, 70, 80, 90, 200, 300, 1000), hergestellt durch ein Verfahren umfassend:
das Verfahren nach einem der Ansprüche 1 bis 14.

## Revendications

1. Procédé pour former des plis (30a-30k) dans un stent-greffe (1, 70, 80, 90, 200, 300, 1000), comprenant :
la formation de plis (30a-30k) dans un matériau de greffage du stent-greffe (1, 70, 80, 90, 200, 300, 1000) en comprimant le stent-greffe (1, 70, 80, 90, 200, 300, 1000) ;
l'application de chaleur au stent-greffe (1, 70, 80, 90, 200, 300, 1000) pour fixer thermiquement les plis (30a-30k) dans le matériau de stent-greffe ; et
**caractérisé en ce qu'**il comprend le retrait axial du stent-greffe jusqu'à sa longueur d'origine pour décompresser le stent-greffe (1, 70, 80, 90, 200, 300, 1000) après que les plis (30a-30k) soient thermiquement fixés.

2. Procédé selon la revendication 1, dans lequel l'application de la chaleur comprend l'application d'un fer à repasser (50) sur les plis des plis (30a-30k) dans le matériau greffé.

3. Procédé selon la revendication 2, dans lequel le fer (50) est chauffé entre 320 °C et 390 °C avant d'appliquer le fer (50) sur les plis.

4. Procédé selon l'une quelconque des revendications 1 à 3, dans lequel la compression du stent-greffe (1) comprend la compression axiale, et éventuellement la compression circulaire, du stent-greffe (1, 70, 80, 90, 200, 300, 1000).

5. Procédé selon l'une des revendications 1 et 4, dans lequel l'application de chaleur comprend la cuisson du stent-greffe (1, 70, 80, 90, 200, 300, 1000) dans un four pendant une période de temps prédéterminée après la formation des plis (30a -30k).

6. Procédé selon la revendication 5, dans lequel le four est chauffé à 320 °C avant la cuisson du stent-greffe (1, 70, 80, 90, 200, 300, 1000).

7. Procédé selon l'une des revendications 5 ou 6, dans lequel la période de temps prédéterminée est de 5 minutes.

8. Procédé selon l'une des revendications 5 ou 6, dans lequel la période de temps prédéterminée est supérieure à 5 minutes.

9. Procédé selon l'une quelconque des revendications 1 à 8, dans lequel la formation des plis (30a à 30k) comprend le pliage du matériau de greffage sur une partie adjacente du matériau de greffage.

10. Procédé selon la revendication 9, dans lequel le pliage du matériau de greffage comprend le pliage du matériau de greffage de manière abluminale par rapport à une lumière du stent-greffe (70).

11. Procédé selon la revendication 10, dans lequel le pliage du matériau de greffage comprend le pliage du matériau de greffage de manière adluminale par rapport à une lumière du stent-greffe (80).

12. Procédé selon l'une quelconque des revendications 1 à 8, dans lequel la formation des plis (30a à 30k) comprend :
le pliage du matériau de greffage de manière adluminale par rapport à une lumière du stent-greffe (90) au niveau d'une partie du matériau de greffage ; et
le pliage du matériau de greffage de manière abluminale par rapport à la lumière du stent-greffe (90) au niveau d'une partie différente du matériau de greffage.

13. Procédé selon l'une quelconque des revendications 1 à 8, dans lequel la formation des plis (30a à 30k) comprend le pliage du matériau de greffage sur une partie adjacente du matériau de greffage pour avoir un espace de greffage (215, 315) entre les éléments de stent-greffe adjacents qui est plus grand sur un côté à plus grande courbure (205, 305) du stent-greffe (200, 300) que sur un côté à moindre courbure (210, 310) du stent-greffe (200, 300).

14. Procédé selon la revendication 13, comprenant en outre l'augmentation de l'espace de greffage sur le côté de plus grande courbure pour diminuer un rayon de courbure du stent-greffe (1).

15. Stent-greffe (1, 70, 80, 90, 200, 300, 1000) fabriqué par un procédé comprenant :
le procédé selon l'une quelconque des revendications 1 à 14.
